(19) Europäisches Patentamt
European Patent Office
Office européen des brevets

(11) EP 2 818 105 B1

## (12) EUROPEAN PATENT SPECIFICATION

(45) Date of publication and mention
of the grant of the patent:
**08.07.2020 Bulletin 2020/28**

(51) Int Cl.:
*A61B 5/08* (2006.01)    *A61B 5/00* (2006.01)

(21) Application number: **14172689.3**

(22) Date of filing: **17.06.2014**

(54) **Apparatus and method for the mobile determination of a physiological stress threshold value**

Vorrichtung und Verfahren zum mobilen Ermitteln eines physiologischen Belastungsschwellwerts

Appareil et procédé pour la détermination mobile d'une valeur de seuil de stress physiologique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.06.2013 DE 102013211908**

(43) Date of publication of application:
**31.12.2014 Bulletin 2015/01**

(73) Proprietor: **Adidas AG**
**91074 Herzogenaurach (DE)**

(72) Inventors:
• **Armitage, Roger**
**Gloucestershire, Cirencester GL7 1FA (GB)**
• **Heyde, Christian**
**79117 Freiburg (DE)**

(74) Representative: **Wegner, Hans et al**
**Bardehle Pagenberg Partnerschaft mbB**
**Patentanwälte, Rechtsanwälte**
**Prinzregentenplatz 7**
**81675 München (DE)**

(56) References cited:
WO-A1-2014/083079     FR-A1- 2 821 262
US-A- 5 810 722       US-A1- 2010 274 100

• KARA M; GÖKBEL H; BEDIZ C S: "A combined method for estimating ventilatory threshold", THE JOURNAL OF SPORTS MEDICINE AND PHYSICAL FITNESS, [Online] vol. 39, no. 1, 31 March 1999 (1999-03-31) , pages 16-19, XP008171081, Italy
• FREDRIK GUSTAFSSON ED - FREDRIK GUSTAFSSON: "Adaptive Filtering and Change Detection", 1 January 2000 (2000-01-01), ADAPTIVE FILTERING AND CHANGE DETECTION, JOHN WILEY & SONS, LTD, PAGE(S) 1 - 126, XP002478407, ISBN: 978-0-471-49287-0 * page 19 * * page 66 *
• NEDER J A ET AL: "A simplified strategy for the estimation of the exercise ventilatory thresholds", MEDICINE AND SCIENCE IN SPORTS AND EXERCISE, LIPPINCOTT WILLIAMS & WILKINS, BALTIMORE, MD, US , vol. 38, no. 5 1 May 2006 (2006-05-01), pages 1007-1013, XP002721455, ISSN: 0195-9131, DOI: 10.1249/01.MSS.0000218141.90442.6C Retrieved from the Internet: URL:http://vidavalorizada.site.med.br/fmfi les/index.asp/::XPR7Z4::/Artigo%20Ventilom etro%20publicado.pdf [retrieved on 2014-08-07]
• Joseph Hall: "CUSUM: Cummulative Summary", , 25 December 2005 (2005-12-25), XP055501185, Retrieved from the Internet: URL:https://web.archive.org/web/2005122516 0654/https://josephhall.org/cusum.html [retrieved on 2018-08-21]

EP 2 818 105 B1

• G. W. Orr ET AL: "A computer linear regression model to determine ventilatory anaerobic threshold", JOURNAL OF APPLIED PHYSIOLOGY., vol. 52, no. 5, 1 May 1982 (1982-05-01), pages 1349-1352, XP055501193, US ISSN: 8750-7587, DOI: 10.1152/jappl.1982.52.5.1349

## Description

### 1. Technical field

The present invention relates to an apparatus and a method for the mobile determination of at least one physiological stress threshold value of a person.

### 2. Prior art

Physiological stress threshold values deliver important information about the fitness, i.e. the training state, of an athlete. Physiological stress threshold values are usually defined as a prominent change of certain physiological parameters like, for example, the concentration of lactate in the blood (lactic acid) or the concentration ratio of oxygen and carbon dioxide in the exhaled air volume of the athlete with increasing stress intensity. It is assumed that the change in these physiological parameters is combined with a change in energy supply of the muscles of the athlete.

Based on lactate-performance diagnostics, per definition, only a physiologically relevant stress threshold value can be determined at the first significant rise of the lactate performance curve (*Lactate Threshold, LT*) (Beaver et al., 1985). Due to mathematical difficulties in determining the *LT*, in the past, a more accurately determinable and reproducible reference point (*Individual Anaerobic Threshold, IAT*) in the steeper course of the lactate performance curve to the right of the *LT* was looked for (Röcker et al., 1998, and Dickhuth et al., 1999).

By the respiratory gas analysis, a first disproportionate rise of the exhaled carbon dioxide compared to the inhaled oxygen (*Anaerobic Threshold, AT*) can be detected (Beaver et al., 1986). It is assumed that the first disproportionate rise of the exhaled carbon dioxide results as a direct consequence from the buffering of blood lactate (through bicarbonate) and that hence there is a causal link between *LT* and *AT*. A further disproportionate rise of minute ventilation as compared to a constant amount of carbon dioxide of the exhaled air is defined as *Respiratory Compensation Point (RCP)*. This point represents the onset of an inadequate hyperventilation as a consequence of increasing acidosis, caused, inter alia, by the increase of blood lactate concentration.

During a stress below the *AT*, different macronutrients such as sugar (carbohydrates) fatty acids and proteins (amino acid) are primarily metabolized using oxygen. This way of providing energy is also denoted as anaerobic and serves to resynthesize adenosine triphosphate (ATP). ATP is the energy carrier which causes the contraction of the muscle. The energy needed for the muscle contraction is for the most part provided by hydrolysis (absorption of water) of ATP in adenosine diphosphate (ADP) and phosphate. An endurance performance below the *AT* can be maintained for a long time, e.g. during a marathon run.

During a stress above the *AT*, the aerobic energy supply is increasingly supported by anaerobic (i.e. primarily without oxygen) ways of energy supply. In this process, during the forced decomposition of dextrose (glucose) or glycogen (a form of storage of glucose) is transformed into lactate in glycolysis pyruvic acid. This always happens when the pyruvic acid and oxygen processing capacities in the mitochondria are reached or the oxygen supply by the blood is limited. The resulting lactate can be retransformed into pyruvic acid by surrounding muscle fibers with still available capacities and then be metabolized in the mitochondrion over the aerobic way of energy supply. In addition, the resulting lactate is introduced from the muscle into the blood circulation and metabolized in the skeleton muscles, the heart or the brain with free capacities under aerobic conditions or converted back into glucose in the liver.

With increasing stress intensity, more and more lactate passes into the blood, so that an acidosis (hyperacidity) of the whole organism occurs in the further progress. Via buffer mechanisms (alkaline countermeasure) it is tried to counteract the dropping pH-value. The beginning hyperventilation after the *RCP* is of particular importance, since it represents, in its functionality, the upper limit of all buffer mechanisms in the organism.

The endurance performance thus does not only depend on the *AT* and the availability of substrate (full energy storage), but also, depending on the intensity, on the acidity tolerance and the buffer capacity. Also during longer competitions, the capacity of providing, for a short time and temporarily, significantly more energy by means of anaerobic metabolism plays an important role in specific competition situations, e.g. with sprints during a football match.

In the course of developing numerous threshold models, in the German and English-speaking areas, different designations both for ventilatory and lactate thresholds were used which are often confounded with regard to their importance. Innumerable determination methods additionally led to confusion. Generally, in lactate diagnosis, the determination of the *IAT* by calculating the minimum lactate equivalent (also called basic lactate) and an added fixed amount proved to be very practical for evaluating the endurance performance of different athletes and normal persons (Röcker et al., 1998).

The minimum lactate equivalent (lactate/performance) serves as a mathematic tool for determining the first rise of blood lactate concentration (*LT*). In the German-speaking area, the *LT* is also denoted as anaerobic threshold. The LAT calculated therefrom is merely a reference point in the steeper section of the lactate performance curve which shows a higher reproducibility than the *LT* (Dickhuth et al., 1999).

On the basis of previous investigations, it is assumed that the *LT* (from lactate diagnostics) and the *AT* (from the respiratory gas analysis) are in causal connection and occur at approximately the same time (*AT* somewhat delayed). In contrast to lactate diagnostics, from the respiratory gas analysis, also the *RCP* may, in addition, be calculated which, in turn, is in high correlation to *IAT* (Dickhuth et al., 1999). Compared with *AT*, the *RCP* is by far easier to calculate. Since the *AT* and the *RCP* are calculated from the respiratory analysis, these thresholds are often also denoted as ventilatory or respiratory thresholds.

From the information on stress intensity at the *LT* and the stress intensity at the *RCP*, the relative functional buffer capacity (*RFBC*) can be calculated. This provides an individual evaluation of the quality of available compensation mechanisms against the pH-decline caused by a stress acidosis.

It is disadvantageous with the determination of physiological stress threshold values known in the prior art that it very much limits the athlete considerably and is virtually only possible under laboratory conditions. Thus, the determination of the lactate concentration requires a regular (e.g. every three minutes) taking of blood, for example at the earlobe during increasing stress intensity e.g. on a treadmill or an ergometer.

The determination of the ventilator threshold requires the measurement of the respiration gases by means of a spirometer (aeroplethysmorgaph) which analyzes the exhaled air of the athlete. The stress control is often performed by means of an ergometer. During the measurement, the athlete wears a face mask to which a volume sensor for measuring the respired air volume as well as a hose, the so-called suction line, are connected. A part of the exhaled air is guided via the suction line to gas sensors in the aeroplethysmograph where its gas content is analyzed.

The known methods for determining physiological stress threshold values can, therefore, virtually only be performed in a laboratory. The athlete is bound to certain devices like an ergometer or a treadmill and may not freely move. Furthermore, special and complex laboratory equipment is needed for determining the lactate concentration and the respired gas concentration.

Kara et al., "A combined method for estimating ventilatory threshold" is directed to a combined method for estimating ventilatory threshold. In a study ventilatory and gas exchange variables were measured breath by breath using Sensormedics 2900 Metabolic Measurement Cart. A rolling average was used as a smoothing filter for breath-by-breath data. Then, for each 15-sec period, one data point was presented as an average. A CUSUM method was based on the increase observed in the variability of $V_E$ or $\dot{V}CO_2$ versus time. The absolute value of the difference between two consecutive measurements of $\dot{V}_E$ or $\dot{V}CO_2$ was added to the sum of the previous differences to produce the cumulative sum. The CUSUMs were then graphed against time and a distinct increase in the slope of the $\dot{V}_E$ or $\dot{V}CO_2$ graph was then detected. The deflection point was determined by the investigator by eye and taken as the VT.

Gustafsson, "Adaptive filtering and change detection" discusses the CUSUM algorithm.

US 2010/0274100 A1 is directed to systems and methods for monitoring subjects in potential physiological distress.

Neder et al., "A simplified strategy for the estimation of the exercise ventilatory thresholds" analyzes the limits of agreement between exercise ventilatory threshold values (VT1 and VT2) estimated from a combination of pulmonary gas exchange and ventilatory variables (cardiopulmonary exercise testing) and those derived from an alternative approach based on the ventilator response only ($\dot{V}E$, ventilometry).

FR 2 281 262 A1 is directed to a method for determining the state of stress for an individual. In a particular embodiment, changes in respiratory volume as a function of time were measured using a respiratory inductive plethysmograph having two turns of sinusoidal paths, one for the ribs and one for the abdomen. In a preferred embodiment, the inductive coils are integrated into a vest. In an illustrated example, a conversion and recording unit is also incorporated in the vest. The conversion and recording module is intended to be connected to a processing device, realized for example by a micro-computer. The changes in respiratory volume are converted into an electrical signal representative of respiratory cycles. The signal has peaks corresponding to the transition phase between inspiration and expiration of each respiratory cycle. The method consists of modeling the peaks of the signal using a polynomial of degree greater than or equal three and calculating a representative stress score of the stress state of the individual from this modelling.

Therefore, it is the objective of the present invention to provide an apparatus for determining a physiological stress threshold value which is mobile, i.e. which may be carried by the athlete without noteworthy limitation during the exercise of almost any sports activity. The apparatus should furthermore be inexpensive to be used in popular sports. A further aspect of the present invention concerns a corresponding method.

## 3. Summary of the invention

The invention is set out in the appended claims.

According to a first aspect of the present invention, this objective is solved by an apparatus for the mobile determination of at least one physiological stress threshold value of an athlete, wherein the apparatus comprises a sensor for determining the respired air volume at each point in time of a plurality of points in time, and a processing unit, which is configured to compute a sum value for each point in time of the plurality of points in time at least based on the respired air volume of a present point in time and the sum value of a previous point in time, to set the sum value to an initial value, if the previous point in time is not within the plurality of points in time, and to determine the physiological stress threshold value based on the computed sum values.

The apparatus according to the invention has the advantage as compared to known apparatuses that only a sensor for determining the respired, i.e. ex- and / or inhaled air volume and a processing unit are needed. A regular taking of blood for measuring the lactate concentration or gas sensors for measuring the concentration of oxygen and carbon dioxide in the respired air can be done without. The apparatus is thus mobile and may be worn on the body by the athlete, for example.

In the apparatus according to the invention, a sensor determines the respired air volume of the athlete at each point in time of a plurality of points in time. The respired air volume may be the inhaled or exhaled air volume or a value derived from both volumes. A point in time may in the context of the present invention also be a time period during which the respired air volume is determined, for example a time period of ten seconds. The respired air volume could, for example, be averaged over this time period.

The determination of the physiological stress threshold value from the respired air volumes determined by the sensor is made possible by the processing unit according to the invention. This unit measures the respired air volume at certain points in time and computes sum values for each point in time at least based on the respired air volume of a present point in time and the sum value of a previous point in time. By this kind of summation of timely subsequent air volumes, the smallest changes in the respired air volume may be determined and one or more physiological stress threshold values may be determined.

It is a particular advantage of the present invention that the data required for determining the physiological stress thresholds can be calculated independently of any laboratory. In contrast to known methods, the athlete is, for example, not bound to an ergometer or has his blood to be taken at regular intervals. Instead, the athlete may exercise almost any sports activity during the recording of the data.

Finally, due to the apparatus according to the invention, complex and expensive laboratory equipment is not necessary. The sensor used according to the invention can furthermore also record data in real-time and thus be used during normal sports activities, for example. Suitable sensors for determining the respired air volume are for example described in WO 2010 027515 A1 and WO 2006 034291 A2 of Vivometrics Inc. and may be integrated in sports apparel.

Preferably, the apparatus is configured to be worn during a sports activity of the athlete without essentially limiting the athlete in his activity. "Essentially" means that the athlete may perform his sports activity as he is used to, as if he were not carrying the apparatus. With the known determination of, e.g., the blood lactate value, a runner, for example, may not run an arbitrary track alone, but needs a third person (e.g. a sport medicine specialist) regularly taking his blood. Therefore, the blood lactate value is usually measured in a laboratory on a treadmill or ergometer. When measuring the oxygen and carbon dioxide concentration in the respired air, the athlete must wear a face mask, whereby he is essentially limited in his sports activity and not mobile.

Preferably, the sensor is based on the principle of respiratory inductive plethysmography. With this method, the measuring of the respired air volume is performed through meander-shaped electrical conductors which can be arranged in the chest and/or abdomen region and respectively form an electrical coil and are connected to an electrical oscillator. Due to the respiratory motions, the circumference of the chest and abdomen and thus the length of the conductors, the inductance of the coils and finally the oscillation frequency alter. The alteration of the oscillation frequency can be evaluated and permits conclusions to be made with regard to the in- and exhaled air volume. Corresponding sensors are, for example, described in the mentioned WO 2006 034291 A2.

Alternatively, the respired air volume can also be determined by means of a magnetometer or a pressure sensor. A further alternative is the determination of changes in length in the thoracic region. In principle, an optical sensor can also be used for this. The respired air volume can also be determined indirectly by the corresponding processing of other measured values. Basically, however, according to the invention, any sensor can be used which is capable of determining the respired air volume.

Preferably, the sensor is integrated in sports apparel. Further preferred, in addition or alternatively the processing unit is integrated in an article of sportswear. For example, a sensor based on the principle of respiratory inductive plethysmography may be sewn, woven or glued into a running shirt, soccer or bicycle jersey. The athlete does not have to don the sensor and / or processing unit in addition to his sports apparel or fix the sensor and / or processing unit thereon. Also, a sensor integrated in the sports apparel is not so likely to slip out of place and delivers more reliable measurement results.

Preferably, the sensor and the processing unit are connected with each other via at least one electrical link. This may be a cable or an electrically conductive fabric. Alternatively, the sensor and the processing unit are wirelessly connected with each other.

Preferably, the processing unit is a processor. A processor may easily and relatively inexpensively be programmed for determining the physiological stress threshold value or may be configured correspondingly in another way.

Preferably, the processing unit may be worn together with the sensor on the body of the athlete, for example in a sports shirt or an electronic apparatus taken along as, for example, a mobile telephone or a media player. Alternatively, the processing unit may be separated from the athlete and communicate wirelessly, for example, with the sensor. For example, the processing unit might be a personal computer, a notebook or a tablet PC which is monitored by a trainer of the athlete, for example. Preferably, the apparatus comprises an alarm device for outputting an alarm if the determined physiological stress threshold value is reached and / or exceeded. In this way, the athlete is warned if an intensity of stress is reached which does not correspond to his training goal. Depending on the training goal, ventilator stress thresholds are an objective measurement for training control. For example, a training above the anaerobic or ventilatory threshold, respectively, is not optimal for endurance sports and fat burning. The athlete may adapt his stress level accordingly if an alarm occurs. For example, a runner may reduce his speed.

The processing unit is configured to multiply the determined air volumes by times associated with the respective points in time. Such a method step leads to a smoothing of the natural spread of the calculated air volumes and thus to a more reliable prediction. Preferably, the zero-point of time is associated with the earliest point in time of the plurality of points in time.

The sum includes a difference which is based on the respired air volume of the present point in time and the respired air volume of a previous point in time. This method step allows the sensitive detection of the smallest changes in the slope of the respired air volume determined over time.

In each sum a weight value is subtracted from the respective determined sum value. A correspondingly chosen weight value ensures that changes in the slope of the determined air volumes are prominently visible, such that the physiological stress threshold value may be determined most reliably.

Preferably, the weight value is constant in time during the plurality of points in time. For example, the weight value may be the expected average value of the respired air volumes such that deviations from the expected average show up in a particularly prominent manner

The weight value is based on an average value over the plurality of points in time. Temporary deviations from the average value, e.g. when reaching a physiological stress threshold value, are thus recognized most reliably.

In an example not according to the invention, the weight value is the respired air volume of the first point in time. Furthermore, alternatively, the weight value is an average value to be expected. For example, it might be the expected average value of a statistical population of athletes.

The average value is based on the respired air volumes. Air volumes which temporarily exceed or fall below the average air volume, e.g. when reaching a physiological threshold, may therefore be determined very reliably due to the applied summation method.

Preferably, the processing unit is configured to determine the physiological stress threshold value based on a change over time of the sum values. A change over time of the sum values computed according to the invention is a reliable indicator for reaching a physiological stress threshold value.

Preferably, the processing unit is configured to determine the physiological stress threshold value based on at least one regression line. Regression lines allow for the modeling of a trend in the computed sum values. A change in the underlying trend may be used as an indication for reaching a physiological stress threshold value.

Preferably, the processing unit is configured to determine the physiological stress threshold value based on a point of intersection of two regression lines. In this way, a change in the underlying trend and thus the presence of a physiological stress threshold value may be reliably determined.

Preferably, the physiological stress threshold value is one of the ventilatory thresholds. As already mentioned, the second ventilator threshold (RCP) strongly correlates with the IAT. The respiratory compensation point RCP (also designated as respiratory threshold) is above the AT (Anaerobic Threshold) or the LT (Lactate Threshold). According to a common definition, the respiratory compensation point denotes an dis-proportional rise in minute ventilation compared to the steadily increasing release of carbon dioxide. According to another definition, the respiratory compensation point denotes the point where, with increasing stress of the body, a decrease of carbon dioxide concentration in the respired air can be determined.

Alternatively, based on a corresponding y-axial intercept, at the determined stress threshold value, a further stress threshold value can be determined in the evolution of the ventilation data over time, which is around a relative fixed value (e.g. 75%) before the calculated stress threshold value. Said stress threshold value estimates the situation of AT or LT, respectively.

Alternatively, the relative span between the stress threshold values is an estimate of the relative functional buffer capacity RFBC which is relevant for sports with high intermittent stress intensities such as game sports.

The presence of at least one stress threshold value constitutes important information for the athlete. On the one hand, the athlete may determine his stress threshold values by means of the apparatus according to the invention (for example LT or AT, respectively, and IAT and RCP, respectively), determine competition prediction for standardized running routes and compare his performance capacity to that of a comparative population. On the other hand, the apparatus according to the invention may be used for training control, i.e. it may, on the one hand, inform the athlete when the individual ventilator threshold or IAT, respectively, is reached during a training unit, and, on the other hand, give the athlete an opportunity of checking his training success on his own.

The stress thresholds are at a higher stress intensity with trained athletes than with untrained athletes. Therefore, the respiratory compensation point gives an important hint to the training state of an athlete and may therefore be used for training control.

Preferably, the apparatus is configured to determine the at least one physiological stress threshold value in real-time. Thus, the athlete may obtain a feedback during the training relating to his stress intensity and may react when the physiological threshold is exceeded.

A further aspect of the present invention relates to a method for the mobile determination of at least one physiological stress threshold value of an athlete, comprising the steps: a.) determining the respired air volume at each point in time of a plurality of points in time; b.) computing a sum value for each point in time of the plurality of points in time at least based on the respired air volume of a present point in time and a sum value of a previous point in time; c.) setting the sum value to an initial value, if the previous point in time is not within the plurality of points in time; d.) determining the physiological stress threshold value based on the computed sum values.

## 4. Short description of the figures

In the following aspects of the present invention are described in more detail referring to the accompanying figures. These figures show:

Fig. 1:      A schematic illustration of an apparatus according to the invention, which may be used with a sports apparel;

Fig. 2:      A diagram, which shows the time evolution of sum values computed from the respired air volume according to the invention.

Fig. 3:      An embodiment of the present invention;

Fig. 4:      A comparison of competition predictions according to the present invention with competition predictions based on laboratory values.

## 5. Detailed description of preferred embodiments

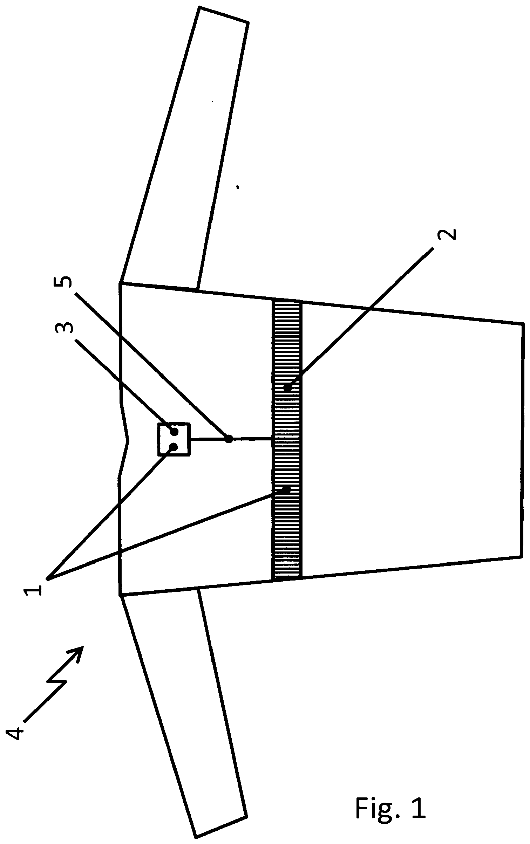

**Fig. 1** shows a presently preferred embodiment of the present invention. In **Fig. 1** an apparatus **1** according to the invention is shown for the mobile determination of at least one physiological stress threshold value of an athlete. The apparatus **1** comprises a sensor **2** for determining the respired air volume at each point in time of a plurality of points in time. The sensor **2** is integrated in a sports apparel **4** in the embodiment of **Fig. 1**. In the preferred embodiment this is a t-shirt, e.g. a running shirt, soccer or bicycle jersey. Basically, the sensor **2** may be separate from the sports apparel.

The sensor **2** may be a sensor which is based on the principle of respiratory inductive plethysmography. With this method, the measuring of the respired air volume is performed through meander-shaped electrical conductors which can be arranged in the chest and/or abdomen region and respectively form an electrical coil and are connected to an electrical oscillator. Due to the respiratory motions, the circumference of the chest and abdomen alter and thus the length of the conductors, the inductance of the coils and finally the oscillation frequency. The alteration of the oscillation frequency can be evaluated and permits conclusions to be made with regard to the in- and exhaled air volume. Corresponding sensors are for example described in the mentioned WO 2006 034291 A2.

The sensor **2** may basically be based on another principle of measuring the respired, i.e. the inhaled and / or exhaled air volume. For example it may be an aeroplethysmograph.

The sensor **2** is configured such that the respired air volume is determined at each point in time of the plurality of points in time. The respired air volume may be the inhaled air volume or the exhaled air volume. Alternatively, the sensor **2** determines the inhaled air volume as well as the exhaled air volume. In this case, it could, for example, determine the inhaled air volume as the average value of the inhaled and exhaled air volume.

The plurality of points in time are at least two different points in time. A point in time is understood in the context of the present invention also as a time period, for example a time period of ten seconds. In this case, the point in time may be defined, for example, as the start point or the end point or the middle point of the time period.

The sensor **2** may determine the respired air volume breath by breath. In this case a point in time is assigned to each breath, where the breath occurred. Since human respiration usually is somewhat irregular, the time differences between the points in time are irregular as well. Alternatively, the sensor **2** may determine the respired air volume for consecutive points in time. For example the respired air volume may be determined over a time period of ten seconds each. In this case, the time differences between the points in time are the same.

The apparatus according to the invention comprises a processing unit **3** which in the exemplary embodiment of **Fig. 1** is fixed to the sports apparel **4**. For example the processing unit **3** may be integrated in the sports apparel, e.g. sewn or glued. Alternatively, the processing unit **3** may be releasably attached to the sports apparel 4, for example by means of at least one push-button or hook-and-loop fastener.

In the exemplary embodiment of **Fig. 1** the processing unit **3** is arranged in the upper back portion of the sports apparel 4. Basically, the processing unit **3** may be arranged at an arbitrary location of a sports apparel or on the body of the athlete.

The processing unit **3** may for example be a processor which is programmed by suitable software. Alternatively, the functions performed by the processing unit **3** are directly integrated in hardware, for example as an application-specific integrated circuit (ASIC) as a field programmable gate array (FPGA).

Basically, the apparatus **1** according to the invention is suitable to be worn during a sports activity of the athlete without essentially limiting the athlete in his activity. "Essentially" means that the athlete may perform his sports activity as he is used to, as if he were not carrying the apparatus 1. For example, the athlete may wear the sports apparel **4** in the exemplary embodiment of **Fig. 1** like any other sports apparel without being limited in his motions.

The processing unit **3** may basically be separated from sports apparel 4. For example, the processing unit **3** may comprise a wrist band an may be worn around a wrist or an upper arm of an athlete. Alternatively, the processing unit **3** may be worn on a belt and may be attached thereto e.g. by a clip. It is furthermore conceivable that the processing unit **3** is integrally formed with the sensor **2**.

In the exemplary embodiment of **Fig. 1**, the processing unit **3** is connected to the sensor **2** by means of an electrical conductor **5**. The electrical conductor **5** may e.g. be integrated in the sports apparel **4** via the electrical conductor **5**. The sensor **2** transmits a signal to the processing unit **3** which corresponds to the respired air volume as determined by the sensor **2**.

Alternatively, the sensor **2** transmits a signal to the processing unit **3** wirelessly, for example by radio. It is conceivable to use Bluetooth or another protocol.

The processing unit uses the air volumes as determined by the sensor **2** to compute a sum value for each point in time of a plurality of points in time, at least based on the respired air volume of a present point in time and a sum value of a previous point in time. For example, the processing unit **3** determines a sum value $S_n$ for a present point in time $n$ with the sum:

$$S_n = S_{n-1} + v_n$$

Here, $S_{n-1}$ is the previous sum value, i.e. the sum value computed at the previous point in time $n$-1 and $v_n$ is the respired air volume of the present point in time $n$, i.e. the respired air volume which is determined by the sensor **2** at the present point in time.

Furthermore, the processing unit **3** is configured to set the sum value to an initial value if the previous point in time is not within the plurality of points in time. For example, if the plurality of points in time comprises the points in time $\{1,2,...,N\}$, the processing unit **3** sets the sum value zero to an initial value $a$:

$$S_0 = a$$

The next sum value $S_1$ computed by the processing unit **3** is then:

$$S_1 = S_0 + x_1 = a + x_1$$

The initial value may for example be zero: $a = 0$. In a presently most preferred embodiment a weight value $w_n$ is subtracted from each determined sum value:

$$S_n = S_{n-1} + v_n - w_n \text{ with } S_0 = a$$

The weight value may be constant over the plurality of points in time.

$w_n \equiv w$ (for all $n$ from the plurality of points in time).

The weight value $w_n$ may be constant in part, i.e. over sections of the plurality of points in time. For example, the weight value in the anaerobic area could take a determined constant, whereas in the anaerobic area, it takes a different constant value.

In case of a constant weight value, it may be an average value which is computed over the plurality of points in time. In a presently particularly preferred embodiment of the invention the average value is based on respired air volumes. The processing unit **3** is configured to compute the average air volume $\bar{v}$ for each point in time of the plurality of points in time:

$$w_n \equiv w = \bar{v} = \frac{1}{N} \sum_{n=1}^{N} v_n$$

Here $N$ is the number of points in time of the plurality of points in time. The processing unit **3** is, therefore, configured in this particularly preferred embodiment to compute the sum values for the point in time $n$ according to the following formula:

$$S_n = S_{n-1} + v_n - \bar{v} \text{ with } S_0 = 0$$

In this case, it became clear that physiological stress threshold values show up very prominently and may be identified very reliably.

Alternatively, the weight value may, for example, be set on the first obtained measurement value: $w_n \equiv x_1$. Other than with the use of the average value $\bar{v}$ as weight value, in this case, not all measurement values have to be known. The process may then be performed in real time.

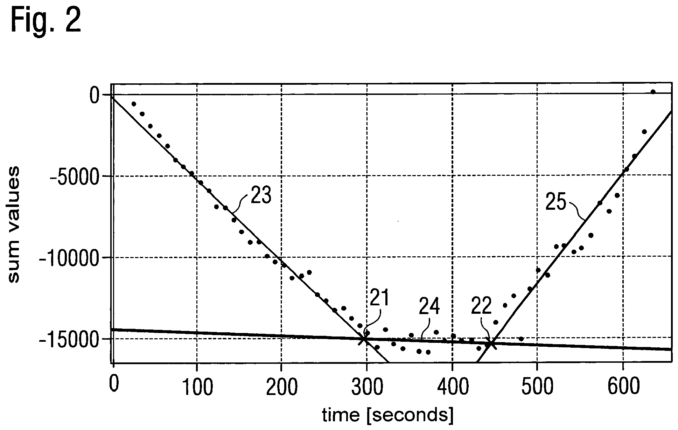

**Fig. 2** shows an exemplary diagram in which the processing unit **3** has computed the sum value according to the above formula $S_n = S_{n-1} + v_n - \bar{v}$. In the diagram of **Fig. 2**, the sum values computed according to the above formula are plotted against the time (in seconds).

The respired air volumes were averaged over time periods of ten seconds each. The initial value was set to zero: $a = 0$. Since the average respired air volume over the plurality of points in time has been subtracted in each summation, the last computed sum value is zero as well: $S_n = 0$.

As can be seen from the diagram of **Fig. 2**, the particularly preferred kind of summation according to the formula above emphasizes temporary deviations from the average respired air volume. Thus, between 0 and 300 seconds, a negative trend is visible, i.e. in this time span the respired air volume was smaller on average than the average air volume $\bar{v}$ respired per point in time over the entire time span of about 600 seconds (i.e. the plurality of points in time).

During the time span of about 300 seconds to 450 seconds, a flat evolution of the computed sum values shows up. In this time span the respired air volume was approximately equal to the average value $\bar{v}$. From about 450 seconds on, the respired air volume is higher than the average value $\bar{v}$.

Summarizing, it results from the diagram of **Fig. 2** that the respired air volume for each point in time has increased over the entire measured time span, i.e. the plurality of points in time. This is in accordance with the continuously increasing stress intensity during the entire time span. To compensate for the increased need of oxygen for the energy supply of the muscles and to remove the generated carbon monoxide, the air volume respired per point in time has increased.

As results from the diagram of **Fig. 2** and as will be explained in the following, this increase of the respired air volume occurs abruptly at certain physiological stress threshold values. The processing unit **3** is therefore configured to determine the at least one physiological stress threshold value based on the computed sum values.

At present, it is particularly preferred that the processing unit **3** determines the physiological stress threshold value based on timely changes of the sum values. For example in the diagram of **Fig. 2**, a prominent change of the computed sum values occurs at about 300 seconds. As initially explained, the respired air volume increases at this point and corresponds approximately to the average air volume $\bar{v}$ of the entire time span of about 600 seconds. This point of the first prominent rise **21** of the respired air volume corresponds well with the ventilator threshold. The point of the second prominent rise **22** of the respired air volume (at about 450 seconds) corresponds well with the respiratory compensation point.

Three regression lines **23**, **24**, **25** are drawn in the diagram of **Fig. 2**. In a particularly preferred embodiment, the processing unit **3** uses regression lines to determine the at least one physiological stress threshold value. For example, a relation to the ventilatory threshold may be determined as the intersection **21** of the first two regression lines **23** and **24**. A relation to the respiratory compensation point may be determined as the intersection **22** of the second regression line **24** and the third regression line **25**.

To determine the location of the regression lines and the intersections, known algorithms may be used. As initial parameter, such an algorithm may, for instance, comprise an area where an intersection is presumed. For example, the first intersection could be located in an area of 0% to 60% of the maximum stress of the athlete.

The apparatus **1** according to the invention may comprise an alarm device (not shown in **Fig. 1**) which outputs an alarm upon reaching and / or exceeding the determined stress threshold value. For example, this may be an acoustical or optical alarm, which outputs a corresponding alarm upon reaching the ventilator threshold **21** and / or the respirator compensation point **22**. In this way, the apparatus **1** according to the invention may be used for training control.

After determining at least one physiological threshold value according to the invention, in the following, this threshold may be used for training control. The athlete then does no longer have to go to his stress limit. The at least one physiological stress threshold value determined according to the invention may be used in a training unit, for example, beside the speed and the heart rate, as threshold value for training goal zones.

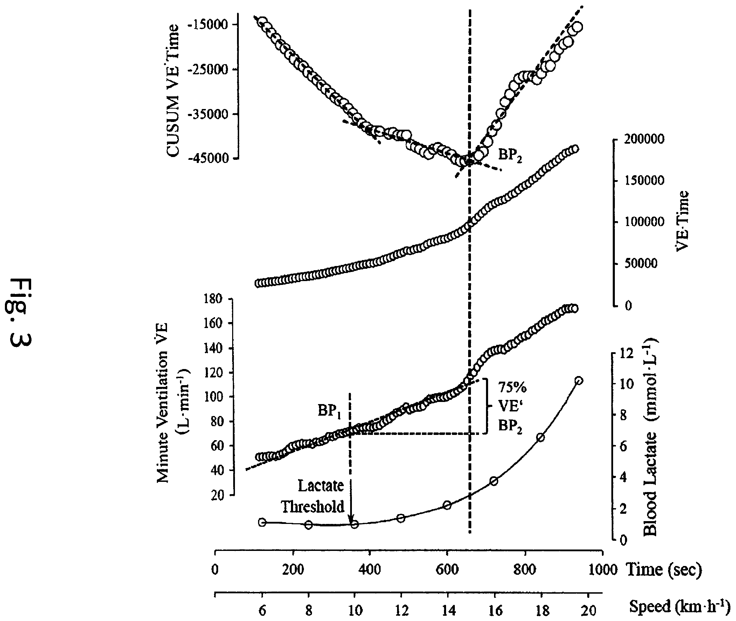

**Fig. 3** shows a further possibility of using the present invention. In the middle third of **Fig. 3**, the respired air volumes determined by means of sensor **2** are plotted against the time or the running speed, respectively, of the athlete. In the upper third of **Fig. 3**, the related respired air volumes processed by a apparatus according to the invention or by use of the method according to the invention, respectively, are plotted. According to the invention, a significant timely change of the respired air volume was determined as transition point $BP_2$. In the example of **Fig. 3**, the transition point $BP_2$ is located at approximately 650 seconds or at a running speed of 15 km/h.

A further point $BP_1$ may now be defined as a reduced relative fixed value before the determined transition point $BP_2$. For example, $BP_1$ could be defined between 50% and 100% or between 65% and 85% of the stress associated with the calculated transition point $BP_2$. In the example of **Fig. 3**, a physiological stress threshold value $BP_1$ is defined at 75% of the stress of $BP_2$. This is at approximately 350 seconds or 10 km/h, respectively, and corresponds well with the significant rise of the lactate performance curve *(Lactate Threshold, LT)*. The lactate curve is shown in the lower third of **Fig. 3** for comparison.

The apparatus according to the invention or the method according to the invention, respectively, may also be used for individual training control, for stress monitoring and for competition prediction. For example, the relative functional buffer capacity *RFBC* can be estimated therefor on the basis of the location of points $BP_1$ and $BP_2$:

$$RFBC_{est} = \frac{BP_2 - BP_1}{BP_2} \cdot 100$$

From the estimated relative functional buffer capacity $RFBC_{est}$, it can, for instance, be estimated how long an athlete will need for a thousand-meter sprint. **BP₁** can then - as shown in connection with **Fig. 3** - be determined as relative percentage stress of **BP₂**. Alternatively, **BP₁** may be determined as the actual transition point - as shown in connection with **Fig. 2.**

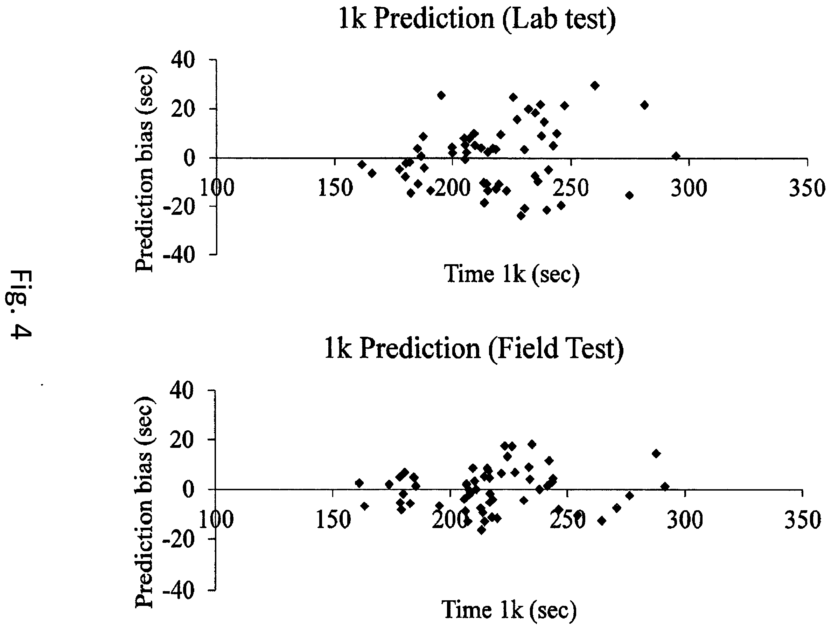

**Fig. 4** shows corresponding estimates of the performance expected for a thousand-meter sprint of 59 athletes. **Fig. 4** shows the deviation of the expected performance from the actual performance in seconds, i.e. the performance was defined as the time needed for a running distance of 1000 m.

For the diagram in the upper half of **Fig. 4**, the performance expected from each athlete was estimated in the laboratory, i.e. by means of lactate diagnostics, the *IAT (Individual Anaerobic Threshold)* was determined. From the *IAT,* the relative functional buffer capacity *RFBC* was calculated, and finally the time expected to be necessary for a distance of 1000 m was determined.

The diagram in the lower half of **Fig. 4** is based on an apparatus according to the invention or a method according to the invention, respectively. First of all, a transition point **BP₂** - as shown in connection with **Fig. 3** - was determined. Then, **BP₁** was determined as the relative fixed value and *RFBC_{est}* was calculated as shown above. Finally, the time expected to be needed for a distance of 1000 m was determined at **BP₂** and *RFBC_{est}* on the basis of the running speed.

From a comparison of the two diagrams in **Fig. 4**, it becomes clear that the values calculated on the basis of the present invention (lower half of **Fig. 4)** present a significantly smaller deviation from the actual performance than the expected values determined on the basis of lactate values (upper half of **Fig. 4**). A statistical analysis shows an average deviation of 6.7 seconds (relative deviation 2.9%, coefficient of determination $R^2$ = 0.92). In contrast thereto, the expected values determined by means of lactate values show an average deviation of 10.6 seconds (relative deviation 4.8%, coefficient of determination $R^2$ = 0.86). The competition prediction determined on the basis of the present invention is thus significantly better than a prediction which is based on lactate values determined in the laboratory.

**Claims**

1. Apparatus (1) for the mobile determination of at least one physiological stress threshold value of an athlete, comprising:

    a. a sensor (2) for determining respired air volume at each point in time of a plurality of points in time;
    b. a processing unit (3), which is configured to:

    Compute a sum value for each point in time of the plurality of points in time at least based on the respired air volume of a present point in time and a sum value of a previous point in time, wherein in each sum a weight value is subtracted from the respective determined sum value, wherein the weight value is based on an average value formed over the plurality of points in time, and wherein the average value is based on the respired air volumes;
    Multiply the determined air volumes with times associated with the respective points in time;
    Set the sum value to an initial value, if the previous point in time is not within the plurality of points in time;
    Determine the physiological stress threshold value based on the computed sum values.

2. Apparatus (1) according to claim 1, wherein the apparatus (1) is configured to be worn during a sports activity of the athlete without essentially limiting the athlete in his activity.

3. Apparatus (1) according to one of the preceding claims, wherein the sensor (2) is based on the principle of respiratory inductive plethysmography.

4. Apparatus (1) according to one of the preceding claims, wherein the sensor (2) is integrated in a sports apparel (4).

5. Apparatus (1) according to one of the preceding claims, wherein the processing unit (3) is integrated in a sports apparel (4).

6. Apparatus (1) according to one of the preceding claims, wherein the processing unit (3) is a processor.

7. Apparatus (1) according to one of the preceding claims, further comprising:
an alarm device for outputting an alarm if the determined physiological stress threshold value is reached and / or exceeded.

8. Apparatus (1) according to claim 1, wherein a zero-point of time is associated with an earliest point in time of the plurality of points in time.

9. Apparatus (1) according to one of the preceding claims, wherein the sum is based on a difference which is based on the respired air volume of the present point in time and the respired air volume of a previous point in time.

10. Apparatus (1) according to claim 1, wherein the weight value is constant in time over the plurality of points in time.

**11.** Apparatus (1) according to one of the preceding claims, wherein the processing unit (3) is configured to determine the physiological stress threshold value based on a change over time of the sum values.

**12.** Apparatus (1) according to one of the preceding claims, wherein the processing unit (3) is configured to determine the physiological stress threshold value based on at least one regression line.

**13.** Apparatus (1) according to one of the preceding claims, wherein the processing unit (3) is configured to determine the physiological stress threshold value based on a point of intersection of two regression lines.

**14.** Apparatus according to one of the preceding claims, wherein the apparatus is configured to determine the at least one physiological stress threshold value in real-time.

**15.** Method for the mobile determination of at least one physiological stress threshold value of an athlete, comprising the steps:

    a. Determining, by a sensor (2), respired air volume at each point in time of a plurality of points in time;

    b. Computing, by a processing unit (3), a sum value for each point in time of the plurality of points in time at least based on the respired air volume of a present point in time and a sum value of a previous point in time, wherein in each sum a weight value is subtracted from the respective determined sum value, wherein the weight value is based on an average value formed over the plurality of points in time, and wherein the average value is based on the respired air volumes;

    c. Multiplying, by the processing unit (3), the determined air volumes with times associated with the respective points in time;

    d. Setting, by the processing unit (3), the sum value to an initial value, if the previous point in time is not within the plurality of points in time;

    e. Determining, by the processing unit (3), the physiological stress threshold value based on the computed sum values.

**Patentansprüche**

**1.** Vorrichtung (1) zum mobilen Ermitteln zumindest eines physiologischen Belastungsschwellwerts eines Sportlers, aufweisend:

    a. einen Sensor (2) zum Ermitteln des geatmeten Luftvolumens zu jedem Zeitpunkt einer Mehrzahl von Zeitpunkten;

    b. eine Verarbeitungseinheit (3), welche eingerichtet ist zum:

        Berechnen jeweils eines Summenwertes für jeden Zeitpunkt aus der Mehrzahl von Zeitpunkten zumindest basierend auf dem geatmeten Luftvolumen eines derzeitigen Zeitpunkts und dem Summenwert eines vorherigen Zeitpunkts, wobei in jeder Summe von dem jeweils ermittelten Summenwert ein Gewichtswert abgezogen wird, wobei der Gewichtswert auf einem über die Mehrzahl von Zeitpunkten gebildeten Durchschnittswert basiert, und wobei der Durchschnittswert auf den geatmeten Luftvolumina basiert;

        Multiplizieren der ermittelten Luftvolumina mit Zeiten, welche den jeweiligen Zeitpunkten zugeordnet sind;

        Setzen des Summenwertes auf einen Anfangswert, falls der vorherige Zeitpunkt nicht innerhalb der Mehrzahl von Zeitpunkten liegt;

        Ermitteln des physiologischen Belastungsschwellwerts basierend auf den berechneten Summenwerten.

**2.** Vorrichtung (1) nach Anspruch 1, wobei die Vorrichtung (1) geeignet ist, während sportlicher Aktivität des Sportlers getragen zu werden, ohne den Sportler in seiner Aktivität wesentlich einzuschränken.

**3.** Vorrichtung (1) nach einem der vorangehenden Ansprüche, wobei der Sensor (2) auf dem Prinzip der respiratorischen induktiven Plethysmographie basiert.

**4.** Vorrichtung (1) nach einem der vorangehenden Ansprüche, wobei der Sensor (2) in einen Sportbekleidungsartikel (4) integriert ist.

**5.** Vorrichtung (1) nach einem der vorangehenden Ansprüche, wobei die Verarbeitungseinheit (3) in einen Sportbekleidungsartikel (4) integriert ist.

**6.** Vorrichtung (1) nach einem der vorangehenden Ansprüche, wobei es sich bei der Verarbeitungseinheit (3) um einen Prozessor handelt.

**7.** Vorrichtung (1) nach einem der vorangehenden Ansprüche, weiter aufweisend: einen Alarmgeber zum Ausgeben eines Alarms beim Erreichen und / oder Überschreiten des ermittelten physiologischen Belastungsschwellwerts.

**8.** Vorrichtung (1) nach Anspruch 1, wobei der Zeitnullpunkt dem frühesten Zeitpunkt der Mehrzahl von Zeitpunkten zugeordnet ist.

9. Vorrichtung (1) nach einem der vorangehenden Ansprüche, wobei die Summe auf einer Differenz basiert, welche auf dem geatmeten Luftvolumen des derzeitigen Zeitpunkts und dem geatmeten Luftvolumen eines vorherigen Zeitpunkts basiert.

10. Vorrichtung (1) nach Anspruch 1, wobei der Gewichtswert über die Mehrzahl von Zeitpunkten zeitlich konstant ist.

11. Vorrichtung (1) nach einem der vorangehenden Ansprüche, wobei die Verarbeitungseinheit (3) eingerichtet ist, den physiologischen Belastungsschwellwert basierend auf einer zeitlichen Änderung der Summenwerte zu ermitteln.

12. Vorrichtung (1) nach einem der vorangehenden Ansprüche, wobei die Verarbeitungseinheit (3) eingerichtet ist, den physiologischen Belastungsschwellwerts basierend auf zumindest einer Regressionsgeraden zu ermitteln.

13. Vorrichtung (1) nach einem der vorangehenden Ansprüche, wobei die Verarbeitungseinheit (3) eingerichtet ist, den physiologischen Belastungsschwellwert basierend auf einem Schnittpunkt zweier Regressionsgeraden zu ermitteln.

14. Vorrichtung (1) nach einem der vorangehenden Ansprüche, wobei die Vorrichtung eingerichtet ist, den zumindest einen physiologischen Belastungsschwellwert in Echtzeit zu ermitteln.

15. Verfahren zum mobilen Ermitteln zumindest eines physiologischen Belastungsschwellwerts eines Sportlers, aufweisend die Schritte:

a. Ermitteln, durch einen Sensor (2), eines geatmeten Luftvolumens zu jedem Zeitpunkt einer Mehrzahl von Zeitpunkten;
b. Berechnen, durch eine Verarbeitungseinheit (3), jeweils eines Summenwertes für jeden Zeitpunkt aus der Mehrzahl von Zeitpunkten zumindest basierend auf dem geatmeten Luftvolumen eines derzeitigen Zeitpunkts und dem Summenwert eines vorherigen Zeitpunkts, wobei in jeder Summe von dem jeweils ermittelten Summenwert ein Gewichtswert abgezogen wird, wobei der Gewichtswert auf einem über die Mehrzahl von Zeitpunkten gebildeten Durchschnittswert basiert, und wobei der Durchschnittswert auf den geatmeten Luftvolumina basiert;
c. Multiplizieren, durch die Verarbeitungseinheit (3), der ermittelten Luftvolumina mit Zeiten, welche den jeweiligen Zeitpunkten zugeordnet sind;

d. Setzen, durch die Verarbeitungseinheit (3), des Summenwertes auf einen Anfangswert, falls der vorherige Zeitpunkt nicht innerhalb der Mehrzahl von Zeitpunkten liegt;
e. Ermitteln, durch die Verarbeitungseinheit (3), des physiologischen Belastungsschwellwerts basierend auf den berechneten Summenwerten.

**Revendications**

1. Appareil (1) destiné à la détermination mobile d'au moins une valeur de seuil de stress physiologique d'un athlète, comprenant :

a. un capteur (2) pour la détermination du volume d'air respiré à chaque instant d'une pluralité d'instants ;
b. une unité de traitement (3), qui est configurée pour :

calculer une valeur de somme pour chaque instant de la pluralité d'instants au moins sur la base du volume d'air respiré à un instant présent et d'une valeur de somme à un instant antérieur, où dans chaque somme une valeur de pondération est soustraite de la valeur de somme déterminée respective, la valeur de pondération étant basée sur une valeur moyenne formée sur la pluralité d'instants, et la valeur moyenne étant basée sur les volumes d'air respiré ;
multiplier les volumes d'air déterminés par des temps associés aux instants respectifs ;
paramétrer la valeur de somme à une valeur initiale, si l'instant antérieur ne fait pas partie de la pluralité d'instants ;
déterminer la valeur de seuil de stress physiologique sur la base des valeurs de somme calculées.

2. Appareil (1) selon la revendication 1, dans lequel l'appareil est configuré pour être porté pendant une activité sportive de l'athlète sans essentiellement limiter l'athlète dans son activité.

3. Appareil (1) selon l'une des revendications précédentes, dans lequel le capteur (2) est basé sur le principe d'une pléthysmographie inductive respiratoire.

4. Appareil (1) selon l'une des revendications précédentes, dans lequel le capteur (2) est intégré à un vêtement de sport (4).

5. Appareil (1) selon l'une des revendications précédentes, dans lequel l'unité de traitement est intégrée à un vêtement de sport (4).

6. Appareil (1) selon l'une des revendications précédentes, dans lequel l'unité de traitement (3) est un processeur.

7. Appareil (1) selon l'une des revendications précédentes, comprenant en outre :
un dispositif d'alarme pour délivrer une alarme si la valeur de seuil de stress physiologique déterminée est atteinte et/ou dépassée.

8. Appareil (1) selon la revendication 1, dans lequel un instant zéro est associé à un instant le plus ancien de la pluralité d'instants.

9. Appareil (1) selon l'une des revendications précédentes, dans lequel la somme est basée sur une différence qui est basée sur le volume d'air respiré de l'instant présent et le volume d'air respiré d'un instant antérieur.

10. Appareil (1) selon la revendication 1, dans lequel la valeur de pondération est constante dans le temps sur la pluralité d'instants.

11. Appareil (1) selon l'une des revendications précédentes, dans lequel l'unité de traitement (3) est configurée pour déterminer la valeur de seuil de stress physiologique sur la base d'une variation dans le temps des valeurs de somme.

12. Appareil (1) selon l'une des revendications précédentes, dans lequel l'unité de traitement (3) est configurée pour déterminer la valeur de seuil de stress physiologique sur la base d'au moins une droite de régression.

13. Appareil (1) selon l'une des revendications précédentes, dans lequel l'unité de traitement (3) est configurée pour déterminer la valeur de seuil de stress physiologique sur la base d'un point d'intersection de deux droites de régression.

14. Appareil (1) selon l'une des revendications précédentes, dans lequel l'appareil est configuré pour déterminer en temps réel l'au moins une valeur de seuil de stress physiologique.

15. Procédé de détermination mobile d'au moins une valeur de seuil de stress physiologique d'un athlète, comprenant les étapes de :

   a. détermination, par un capteur (2), du volume d'air respiré à chaque instant d'une pluralité d'instants ;
   b. calcul, par l'unité de traitement, d'une valeur de somme pour chaque instant de la pluralité d'instants au moins sur la base du volume d'air respiré d'un instant présent et d'une valeur de somme d'un instant antérieur, où dans chaque somme une valeur de pondération est soustraite de la valeur de somme déterminée respective, la valeur de pondération étant basée sur une valeur moyenne formée sur la pluralité d'instants, et la valeur moyenne étant basée sur les volumes d'air respirés ;
   c. la multiplication, par l'unité de traitement (3), des volumes d'air déterminés par des temps associés aux instants respectifs ;
   d. le paramétrage, par l'unité de traitement (3), de la valeur de somme à une valeur initiale, si l'instant antérieur ne fait pas partie de la pluralité d'instants ;
   e. la détermination, par l'unité de traitement (3), de la valeur de seuil de stress physiologique sur la base des valeurs de somme calculées.

Fig. 1

Fig. 2

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100274100 A1
- FR 2281262 A1
- WO 2010027515 A1
- WO 2006034291 A2